# EUROPÄISCHE PATENTSCHRIFT

(11) **EP 3 448 154 B1**
(45) Veröffentlichungstag und Bekanntmachung des Hinweises auf die Patenterteilung: **17.03.2021**
(21) Anmeldenummer: 17719155.8
(22) Anmeldetag: 31.03.2017
(51) Int. Cl.: A01N 1/02, G01K 11/02

(54) **VORRICHTUNG UND VERFAHREN ZUR TEMPERATURÜBERWACHUNG EINER KRYOKONSERVIERTEN BIOLOGISCHEN PROBE**
DEVICE AND METHOD FOR MONITORING THE TEMPERATURE OF A CRYOGENICALLY PRESERVED BIOLOGICAL SAMPLE
DISPOSITIF ET PROCÉDÉ DE SURVEILLANCE DE LA TEMPÉRATURE D'UN ÉCHANTILLON BIOLOGIQUE CRYOCONSERVÉ

(30) Priorität: 27.04.2016 DE 102016005078
(43) Veröffentlichungstag der Anmeldung: 06.03.2019
(73) Patentinhaber: Fraunhofer-Gesellschaft zur Förderung der angewandten Forschung e.V., 80686 München (DE)
(72) Erfinder: FUHR, Günter R., 13187 Berlin (DE); ZIMMERMANN, Heiko, 97295 Waldbrunn (DE)
(74) Vertreter: v. Bezold & Partner Patentanwälte - PartG mbB
(86) Internationale Anmeldenummer: PCT/EP2017/000402
(87) Internationale Veröffentlichungsnummer: WO 2017/186329

(56) Entgegenhaltungen:
- EP-A1- 2 937 850
- WO-A1-2007/085385
- JP-A- 2008 151 716
- US-A- 3 701 282
- US-A1- 2006 078 036
- Biocision: "Snap Freezing Using Dry Ice or Liquid Nitrogen", , 1 January 2014 (2014-01-01), XP055647966, Retrieved from the Internet: URL:http://www.biocision.com/uploads/docs/ appnote_snapfreezing_2013.pdf [retrieved on 2019-11-29]
- Christa K Goodell ET AL: "Ring test evaluation of the detection of influenza A virus in swine oral fluids by real-time reverse-transcription polymerase chain reaction and virus isolation", CANADIAN JOURNAL OF VETERINARY RESEARCH., vol. 80, no. 1, 1 January 2016 (2016-01-01), pages 12-20, XP055647969, CA ISSN: 0830-9000

## Beschreibung

Die Erfindung betrifft eine Vorrichtung zur Temperaturüberwachung einer kryokonservierten biologischen Probe. Die Erfindung betrifft ferner ein Verfahren zur Temperaturüberwachung einer kryokonservierten biologischen Probe.

Die Tieftemperaturkonservierung (Kryokonservierung) von Zellen ist bisher die einzige Möglichkeit, Lebensprozesse auf zellulärer Ebene reversibel (vitalitätserhaltend) so anzuhalten, dass sie nach einer Erwärmung auf physiologische Temperaturen wieder anlaufen können. Die Kryokonservierung hat sich über große Biobanken in den letzten Jahrzehnten zu einem unverzichtbaren Element für Kliniken, Pharmaunternehmen, die Arterhaltung, den Umweltschutz und die Gesundheitsvorsorge entwickelt. Gelagert wird biologisches Material in tieftemperaturverträglichen Probenbehältern (Kryobehältern), z. B. Röhrchen, Straws und Beuteln, unterschiedlicher Größe. Bei der Kryokonservierung sind die gelagerten Biomaterialien unter Aufrechterhaltung der Vitalität des Probenmaterials gefroren, zumeist bei Temperaturen unterhalb -80 °C, für Lebendsammlungen unter -140 °C bis zur Temperatur des flüssigen Stickstoffs. Für eine kryokonservierte Probe oder eine für die Kryokonservierung vorgesehene Probe wird nachfolgend auch der Begriff "Kryoprobe" verwendet.

Für makroskopische Proben, wie z. B. Blut oder Gewebe, sind zahlreiche Techniken zur Probenlagerung bei tiefen Temperaturen entwickelt worden. In der modernen Medizin, Gentechnik und Biologie besteht die Tendenz, zunehmend kleine Proben einer Kryokonservierung zu unterziehen. Es werden beispielsweise kleine Suspensionsvolumina (Milliliter oder darunter) mit suspendierten Zellen oder Zellgruppen eingefroren. Die Kryokonservierung von Zellen aus In-vitro-Kulturen erfolgt in überwiegendem Maße in einer Suspension. Die meisten der biomedizinisch relevanten Zellen benötigen jedoch zu ihrer Vermehrung und geordneten Entwicklung einen Substratkontakt. Daher werden Proben ggf. nach einer Kultivierung im substratgebundenen Zustand eingefroren.

Die Qualität der Proben ist von ausschlaggebender Bedeutung, da sie für Zelltherapien in Kliniken, die Entwicklung von Pharmaka und biotechnologischen Produkten, als nationale Ressourcen und vieles mehr Anwendung finden. Die Lagerzeit liegt bei einigen Tagen bis zu Jahrzehnten, mit einer Tendenz zur Langzeitlagerung. Die Proben werden in gekühlten Behältern gelagert, befinden sich zumeist in Metalleinschüben und Racks, mit denen sie bei neuen Einlagerungen oder Entnahmen Temperaturschwankungen unterliegen. Bei Lebendablagen (Zellen, Zellsuspensionen und Gewebeteilen) spielt nicht nur die ununterbrochene Kühlkette eine entscheidende Rolle, sondern auch die Vermeidung großer Temperatursprünge in der Tiefkühlphase. Da es bei der Entnahme gar nicht so selten vorkommt, dass Kryobehälter sich auf Temperaturen von -80 °C bis -20 °C erwärmen, treten, obwohl sie noch gefroren sind, Qualitätsminderungen unerkannt auf, die nicht nur den Wert der Probe mindern, sondern auch bei ihrer Verwendung im klinischen Bereich zu lebensgefährlichen Situationen führen können. Selbst kurzzeitig aufgetauten Proben sieht man im wiedergefrorenen Zustand nicht an, dass sie dem Originalzustand nicht mehr entsprechen. Es geht aber vornehmlich nicht nur darum, ein Auftauen der Biomaterialien zu erkennen, sondern das Überschreiten einer Grenztemperatur im Bereich zwischen -140 °C und -20 °C zu dokumentieren. Eine Temperaturkontrolle und -dokumentation für jede Probe ist die Forderung und bislang nur selten - und wenn, dann mit hohem technischen Aufwand - zu erfüllen. Hinzu kommen umfangreiche Laboruntersuchungen nach dem Auftauen, die ebenfalls wertvolles Probenmaterial verbrauchen und selbst im Falle inzwischen wertlos gewordener Kryoproben Kosten erzeugen.

WO 2007/085385 A1 offenbart einen Probenträger zur Kryokonservierung biologischer Proben. Ein Deckelteil kann in einem Stapel von Probenträgern einen Abschluss oder ein Zwischenstück bilden, wobei ein Kontrollelement in das Deckelteil integriert sein kann, welches eine Erwärmung durch eine Strukturänderung oder einen Farbumschlag anzeigt.

Es ist somit eine Aufgabe der Erfindung, eine Vorrichtung zur Temperaturüberwachung einer kryokonservierten biologischen Probe bereitzustellen, mit der Nachteile herkömmlicher Techniken vermieden werden können. Eine weitere Aufgabe ist es, ein verbessertes Verfahren zur Temperaturüberwachung einer kryokonservierten biologischen Probe bereitzustellen, mit dem Nachteile herkömmlicher Techniken vermieden werden können und das sich durch eine vereinfachte Verfahrensführung auszeichnet.

Eine weitere Aufgabe ist es, eine Möglichkeit bereitzustellen, um an einem möglichst einfachen Marker oder Kennzeichen erkennen zu können, ob eine Kryoprobe sich über eine definierbare Grenztemperatur, und wenn auch nur kurzzeitig, erwärmt hat. Die Grenztemperatur muss im Bereich zwischen -20 °C und -140 °C vor dem Einfrieren festlegbar sein. Dies sollte an jeder einzelnen Kryoprobe und an damit Millionen von Proben rasch und leicht erkennbar möglich sein, darf die Biomaterialien nicht verändern und sollte bereits im tiefgefrorenen Zustand erfolgen. Wenn möglich, sollte der Zustand der Probe auch im Lagerbehälter erfassbar sein, da jede Aus- und Einlagerung die Gefahr der Probenveränderung einer Vielzahl von Proben im Lagergut mit sich bringt, da in der Regel ganze Racks aufgezogen werden. Die Vorrichtung bzw. das Verfahren sollte leicht handhabbar, tieftemperaturtolerant und einstellbar sein. Es darf nur wenig oder keine Energie verbrauchen und möglichst nur geringste Kosten verursachen, da die Lagerung einer Bioprobe im gekühlten Zustand in ihren Gesamtaufwendungen nur wenige Euros kosten sollte. Diesem Anspruch müssen auch die einsetzbaren Materialien gerecht werden. Es wäre weiterhin wünschenswert, wenn nicht nur die Überschreitung einer zu überwachenden Grenztemperatur, sondern auch ein Maß für die Zeitdauer dieser Überschreitung erfasst werden könnte.

Diese Aufgaben werden durch Vorrichtungen und Verfahren mit den Merkmalen der unabhängigen Ansprüche gelöst. Vorteilhafte Ausführungsformen und Anwendungen der Erfindung ergeben sich aus den abhängigen Ansprüchen und werden in der folgenden Beschreibung unter teilweiser Bezugnahme auf die Figuren näher erläutert.

Gemäß einem ersten Gesichtspunkt der Erfindung werden die genannten Aufgaben durch eine Vorrichtung zur Temperaturüberwachung einer kryokonservierten biologischen Probe gelöst. Die Vorrichtung umfasst einen Probenbehälter mit einem Aufnahmeraum (Probenreservoir) zur Aufnahme einer biologischen Probe. Der Probenbehälter ist insbesondere ein Kryoprobenbehälter. Bei der Verwendung der Vorrichtung kann der Aufnahmehohlraum eine kryokonservierte Probe enthalten.

Die Vorrichtung umfasst ferner eine Kammer, deren Innenraum mit dem Aufnahmeraum nicht fluidisch verbunden ist und zumindest zum Teil mit einer Indikatorsubstanz gefüllt ist, deren Siedetemperatur oder Sublimationstemperatur in einem Bereich von -10 °C bis-140 °C liegt. Bei der Siedetemperatur kann es sich um die Normalsiedetemperatur, also der Siedetemperatur bei Normaldruck (1013,25 hPa) handeln. Bei der Sublimationstemperatur kann es sich um die Normalsublimationstemperatur, also der Sublimationstemperatur bei Normaldruck (1013,25 hPa) handeln. Die Kammer weist mindestens eine Öffnung auf, über die die Indikatorsubstanz bei Überschreitung ihres Siedepunkts oder ihres Sublimationspunkts aus dem Innenraum der Kammer entweichen kann. Die mindestens eine Öffnung oder die Kammer sind so angeordnet, dass die Indikatorsubstanz im flüssigen oder festen Zustand nicht über die Öffnung aus der Kammer entweichen kann. Die Öffnung kann beispielsweise über eine Kapillare, beispielsweise eine gebogene Kapillare, gebildet sein, über die der Innenraum mit der Umgebung fluidisch in Verbindung steht. Die Vorrichtung kann auch mehrere derartige Kammern umfassen.

Durch die Kammer der erfindungsgemäßen Vorrichtung wird somit ein Zusatzkompartiment bereitgestellt, das mit einer Flüssigkeit oder einem Festkörper, die oder der bei einer Grenztemperatur zwischen -140 °C und -10 °C seinen Siede- oder Sublimationspunkt besitzt, als Indikatorsubstanz gefüllt ist. Über die mindestens eine Öffnung der Kammer kann die Indikatorsubstanz nur im gasförmigen Aggregatszustand entweichen, d. h., der Füllstand in der als Testvolumen dienenden Kammer nimmt durch das Sieden oder Sublimieren der Indikatorsubstanz ab. Wird eine zu überwachende Grenztemperatur, die der Siedetemperatur oder der Sublimationstemperatur der Indikatorsubstanz entspricht, überschritten, wird die Indikatorsubstanz gasförmig und kann über die mindestens eine Öffnung aus dem Innenraum der Kammer entweichen. Wird somit zu einem späteren Kontrollzeitpunkt festgestellt, dass die Menge an fester oder flüssiger Indikatorsubstanz in der Kammer abgenommen hat, kann auf eine zwischenzeitlich erfolgte, zumindest kurzzeitige Überschreitung der Grenztemperatur geschlossen werden. Die Kammer kann daher als Indikatorelement bzw. Indikatoreinrichtung verwendet werden, um eine unerwünschte Überschreitung der Grenztemperatur anzuzeigen.

Ein besonderer Vorzug der erfindungsgemäßen Vorrichtung ist ferner, dass aus der Menge an Indikatorsubstanz, die sich zu einem bestimmten Zeitpunkt noch in der Kammer befindet, ein Maß für die Zeitdauer einer zurückliegenden Überschreitung der Siedetemperatur oder der Sublimationstemperatur abgeleitet werden kann. Zwar ist die Quantität der Abnahme nicht nur von der Dauer, sondern auch von der Höhe der Temperaturüberschreitung abhängig, gibt aber dennoch einen Hinweis darauf, ob es sich um eine eventuell tolerierbare kurze Überschreitung oder einen längeren Zeitraum gehandelt hat.

Beispielhaft lassen sich folgende Substanzen nennen, die als Indikatorsubstanz verwendet werden können und die in kleinen Mengen auch ohne Probleme in die Raumatmosphäre entlassen werden können: Lachgas (N₂O) (Siedepunkt: - 88,46 °C, Schmelzpunkt: - 90,86 °C), Trockeneis (CO₂) (Sublimationspunkt:- 78,50 °C) oder Ammoniak (NH₃) (Siedepunkt: - 33,35 °C, Schmelzpunkt: -77,72 °C). Einschlägigen chemischen Tabellen lassen sich eine Reihe von weiteren Verbindungen mit geeigneten Siedepunkten oder Sublimationspunkten entnehmen, die sich ebenfalls für den genannten Zweck eignen. Als Indikatorsubstanz kann somit zweckmäßig eine Substanz ausgewählt werden, deren Siedepunkt oder Sublimationspunkt einer vorbestimmten Grenztemperatur, deren Überschreiten überwacht werden soll, entspricht oder zumindest nahe kommt.

Zur besseren Erkennbarkeit kann die Indikatorsubstanz einen Indikatorzusatz enthalten, der eine Detektierbarkeit einer physikalischen Eigenschaft der Indikatorsubstanz erhöht. Der Indikatorzusatz kann beispielsweise ein Farbstoff sein, so dass die Indikatorsubstanz farbig oder gefärbt, d. h. nicht transparent, ist. Die Menge an Indikatorsubstanz, die sich zu einem Überprüfungszeitpunkt noch in der Kammer befindet, kann dann einfacher bestimmt werden. Der Indikatorzusatz können Partikel, insbesondere Nanopartikel sein, die eine Streuwirkung und/oder Polarisationswirkung der Indikatorsubstanz für auf die Indikatorsubstanz auftreffende elektromagnetische Strahlung erhöhen. Dadurch kann eine Menge an Indikatorsubstanz, die sich zu einem Überprüfungszeitpunkt noch in der Kammer befindet, mittels einer optischen Transmissionsmessung, Streumessung und/oder Polarisationsmessung zuverlässiger detektiert werden. Der Indikatorzusatz können leitfähige Partikel sein. Durch Beimischen von leitfähigen Partikel kann die Leitfähigkeit oder Impedanz der Indikatorsubstanz beeinflusst werden. Auf diese Weise kann eine Konfigurationsänderung der Indikatorsubstanz mittels einer Leitfähigkeitsmessung oder Impendanzmessung detektiert werden. Aufgrund des beigemischten Indikatorzusatzes kann die entsprechende Eigenschaft der Indikatorsubstanz mit einer entsprechend zweckmäßig ausgebildeten Messeinrichtung erfasst werden, um einen Füllstand der Indikatorsubstanz in der Kammer zuverlässiger bestimmen zu können.

Gemäß einer weiteren bevorzugten Ausführungsform kann die Vorrichtung eine Mehrzahl von Kammern aufweisen, die jeweils zumindest zum Teil mit einer Indikatorsubstanz, deren Siedetemperatur oder Sublimationstemperatur in einem Bereich von -10 °C bis -140 °C liegt, gefüllt sind, wobei die Indikatorsubstanzen in den Kammern unterschiedliche Siedetemperaturen oder Sublimationstemperaturen aufweisen.

Beispielsweise kann ein Behälter zur Ausbildung mehrerer Kammern mehrere durch Trennwände gebildete Teilhohlräume aufweisen. Hierbei bildet jeder Teilhohlraum eine Kammer aus, die eine der Indikatorsubstanzen enthält. Die Indikatorsubstanzen in den Kammern können unterschiedliche Siede- oder Sublimationstemperaturen aufweisen. Damit können unterschiedliche Temperaturgrenzwerte überwacht werden, wobei jede Indikatorsubstanz so ausgewählt ist, dass ihre Siedetemperatur oder Sublimationstemperatur einem der zu überwachenden Temperaturgrenzwerte entspricht oder zumindest nahe kommt. Diese Ausführungsform bietet den Vorteil, dass sich die erreichten Temperaturintervalle, in die die Probe gelangt ist, genauer eingrenzen lassen.

Ferner kann eine Wandung der mindestens einen Kammer an mindestens einer Stelle transparent oder semi-transparent ausgeführt sein, so dass der Füllstand der Kammer von außen beobachtbar ist.

Ferner ist es vorteilhaft, wenn eine Wandung der mindestens einen Kammer eine Skala zur Anzeige eines Füllstands der Indikatorsubstanz im Innenraum und/oder eine Skala zur Anzeige einer Zeitdauer der Überschreitung des Siedepunkts oder des Sublimationspunkts der Indikatorsubstanz aufweist. Der Zusammenhang zwischen Füllstand der Kammer und Zeitdauer der Überschreitung des Siedepunkts oder des Sublimationspunkts kann beispielsweise vorab experimentell geprüft werden. Diese Variante eignet sich besonders für die visuelle Sichtprüfung.

Gemäß einem weiteren Aspekt der Erfindung kann die Vorrichtung eine Messeinrichtung umfassen, die ausgebildet ist, einen Füllstand der Indikatorsubstanz im Innenraum der mindestens einen Kammer zu bestimmen. Diese Variante eignet sich besonders für die automatisierte Temperaturüberwachung von Kryoproben. Die Messeinrichtung kann lediglich beispielhaft eine optische oder optisch-elektrische Messeinrichtung sein, um z. B. mit einer optischen Transmissions, Streulicht- oder Reflektionsmessung den Füllstand der Indikatorsubstanz festzustellen.

Gemäß einer bevorzugten Ausführungsform kann in einem Teilbereich der Kammer, in den die mindestens eine Öffnung nicht mündet, ein Material mit flüssigkeitsaufnehmender Struktur, beispielsweise ein poröses Material, vorhanden sein.

Diese Ausführungsform ist vorteilhaft bei einer Indikatorsubstanz, die nicht direkt sublimiert, sondern in einem Temperaturbereich unterhalb ihres Siedepunktes flüssig ist, z. B. Lachgas. Im flüssigen Aggregatszustand kann die Indikatorsubstanz dann in das Material mit flüssigkeitsaufnehmender Struktur langsam hineindiffundieren, was quantitativ abgelesen werden kann. Liegt die Lagertemperatur oberhalb der Siedetemperatur, verdunstet das Lachgas über die mindestens eine Öffnung der Kammer. Dann ist die Abnahme des Pegels der Indikatorsubstanz das Maß für die Überschreitung der zu überwachenden Grenztemperatur und auch das poröse Material frei von diesem. Aus diesen Kombinationsbefunden lässt sich weit mehr Information über die Lagertemperatur gewinnen als über ein reines Schmelzen einer Substanz. Zudem ist der Verlust des Materials bei besonderer Zusammensetzung fälschungssicher. In geeigneter Weise kann ein Wiederbefüllen auch unterbunden werden.

Die mindestens eine Kammer kann durch einen Behälter mit einem oder mehreren Hohlräumen gebildet sein, der außen am Probenbehälter anordenbar und/oder angeordnet ist. Der Begriff "anordenbar und/oder angeordnet" soll umfassen "befestigbar und/oder befestigt", "koppelbar und/oder gekoppelt", "verbindbar und/oder verbunden". Der Behälter zur Ausbildung der mindestens einen zumindest zum Teil mit Indikatorsubstanz gefüllten Kammer ist somit von dem Probenbehälter zu unterscheiden.

Der Behälter kann hierbei außen am Behälter anordenbar und/oder angeordnet sein oder im Inneren des Behälters. Eine Möglichkeit der erfindungsgemäßen Realisierung sieht vor, dass der Behälter zur Ausbildung der mindestens einen Kammer lösbar am Probenbehälter befestigt ist. Unter einer lösbaren Befestigung soll insbesondere auch ein Aufschieben oder Aufstecken des Behälters am Probenbehälter umfasst sein. Dies bietet den Vorteil, dass der Behälter räumlich getrennt vom Probenbehälter gelagert und vorbereitet (z. B. Befüllen mit der Indikatorsubstanz) werden kann.

Beispielsweise kann der Probenbehälter einen Deckel zum Verschließen des Aufnahmeraums aufweisen. Gemäß einem bevorzugten Ausführungsbeispiel kann die mindestens eine Kammer in den Deckel, z. B. in das Kopfteil und/oder den Schaft des Deckels, integriert sein. Beispielsweise kann der Deckel einen mit einem oberen Endbereich des Aufnahmeraums des Probenbehälters in Eingriff stehenden Schaft aufweisen, wobei die mindestens eine Kammer in den Schaft integriert ist. Gemäß einer bevorzugten Variante dieses Ausführungsbeispiels ist der Probenbehälter ein Kryoröhrchen, aufweisend einen Deckel zum Verschließen des Aufnahmeraums, der einen mit einem oberen Endbereich des Aufnahmeraums in Eingriff stehenden Schaft aufweist. Hierbei ist die mindestens eine Kammer in den Schaft integriert, und ein auf dem Schaft sitzendes Kopfteil des Deckels weist zur Ausbildung der mindestens einen Öffnung eine Durchgangsöffnung auf, die den Innenraum der Kammer mit der Umgebung verbindet.

Die Anordnung der mindestens einen Kammer im Deckel bietet den besonderen Vorzug, dass kein zusätzlicher Bauraum außerhalb des Probenbehälters vonnöten ist. Ein weiterer Vorteil ist, dass die als Indikatoreinrichtung dienende mindestens eine Kammer zusammen mit dem Deckel räumlich getrennt vom restlichen Probenbehälter gelagert und vorbereitet (z. B. Befüllen der Kammer mit der Indikatorsubstanz und Abkühlen der Indikatorsubstanz, so dass diese fest oder flüssig wird) werden kann.

Eine weitere Möglichkeit der erfindungsgemäßen Realisierung sieht vor, dass die mindestens eine Kammer durch einen Behälter gebildet ist und dass an einer Außenwand des Probenbehälters eine Aufnahme, beispielsweise eine Hülse oder Einstecktasche, befestigt ist, in die der Behälter zur Halterung am Probenbehälter einsteckbar und/oder eingesteckt ist.

Gemäß einer weiteren vorteilhaften Ausführungsform kann die mindestens eine Kammer durch ein doppelwandiges Aufsteckteil gebildet sein, das auf eine äußere Mantelfläche des Probenbehälters aufsteckbar oder aufschiebbar ist und diese im aufgesteckten Zustand dabei zumindest teilweise umgreift. Diese Variante ist besonders vorteilhaft für zylinderförmige Probenbehälter, insbesondere Kryoröhrchen. Das doppelwandige Aufsteckteil kann dabei als Hohlzylinder oder Teil-Hohlzylinder ausgeführt sein, dessen Innendurchmesser dem Außendurchmesser des Probenbehälters entspricht, so dass das Aufsteckteil den zylinderförmigen Probenbehälter manschettenartig oder schellenartig umgreift.

Der Probenbehälter kann ferner mit dem Aufsteckteil verklebt, verschmolzen oder anderweitig fest fixiert sein. Dadurch kann ein Entfernen des Aufsteckteils zu Manipulationszwecken, z. B. um ein Aufsteckteil, das eine unerwünschte Erwärmung anzeigt, durch ein neues Aufsteckteil zu ersetzen, verhindert werden.

Mit dem Begriff Probenbehälter wird insbesondere ein für eine Kryokonservierung ausgelegter Behälter bezeichnet, beispielsweise ein Röhrchen, ein Straw (auch als Samenröhrchen bezeichnet), ein Beutel zur Blut- oder Stammzellenlagerung, eine Box oder ein anderer für eine Kryokonservierung geeigneter Behälter. Derartige Behälter werden entsprechend auch als Kryoröhrchen, Kryostraw, Kryobeutel, Kryobox oder allgemein als Kryobehälter bezeichnet.

Kryoröhrchen (engl. cryogenic tubes) werden auch als Biobank- oder Kryobankröhrchen bezeichnet. Kryoröhrchen weisen einen Aufnahmeraum auf, der einen inneren Hohlraum zur Aufnahme einer biologischen Probe ausbildet. Das Kryoröhrchen weist ferner üblicherweise einen Deckel zum Verschließen des Aufnahmeraums auf. Der Deckel kann einen Eingriff aufweisen, über den der Deckel mit einem Werkzeug gedreht werden kann. Das Kryoröhrchen kann auch ein Bodenelement aufweisen, das eine Kennung, z. B. in Form eines maschinenlesbaren Codes, aufweist.

Der Probenbehälter ist vorzugsweise unter Verwendung tieftemperaturverträglichen Kunststoffmaterials für Temperaturen unter -140 °C hergestellt. Das Kunststoffmaterial kann ohne Veränderung und ohne Schaden wiederholte Temperaturwechsel tolerieren. Es wird vorzugsweise ein Kunststoffmaterial verwendet, dessen Wasseraufnahmefähigkeit < 1 % der Eigenmasse, insbesondere < 0.1 % der Eigenmasse beträgt. Erfindungsgemäße Kryospeicherelemente basieren beispielsweise auf Polyurethan oder Polyethylen. Mit dem Begriff "biologische Probe" wird biologisches Material wie Zellen, Gewebe, Zellbestandteile, biologische Makromoleküle etc. bezeichnet, welches im Probenbehälter der Kryokonservierung unterzogen werden kann - ggf. in einer Suspension und/oder im Verbund mit einem Substratmaterial. Im Aufnahmeraum kann somit ein Substrat angeordnet sein, das zur adhärenten Aufnahme biologischer Zellen, die Teil der biologischen Probe sind, eingerichtet ist.

Gemäß einem zweiten Gesichtspunkt der Erfindung werden die genannten Aufgaben durch ein Verfahren zur Temperaturüberwachung von kryokonservierten Proben gelöst, das eine Vorrichtung zur Temperaturüberwachung, wie in diesem Dokument beschrieben, verwendet. Die Ausführungen betreffend die Vorrichtung, insbesondere deren vorteilhafte Ausführungsvarianten, sollen somit zur Vermeidung von Wiederholungen auch als verfahrensgemäß offenbart gelten.

Als Indikatorsubstanz kann vorzugsweise wiederum eine Substanz ausgewählt werden, deren Siede- oder Sublimationstemperatur einer vorbestimmten Grenztemperatur, deren Überschreiten überwacht werden soll, entspricht oder dieser zumindest nahe kommt.

Gemäß dem Verfahren kann somit eine Vorrichtung zur Temperaturüberwachung, wie in diesem Dokument beschrieben, bereitgestellt werden, wobei die Vorrichtung mindestens eine Indikatorsubstanz im flüssigen oder festen Zustand in der mindestens einen Kammer enthält, wobei die Siedetemperatur oder Sublimationstemperatur der Indikatorsubstanz in einem Bereich von -10 °C bis -140 °C liegt. Die Kammer weist mindestens eine Öffnung auf, über die die Indikatorsubstanz bei Überschreitung ihres Siedepunkts oder ihres Sublimationspunkts aus dem Innenraum der Kammer entweichen kann. Der Aufnahmeraum des Probenbehälters enthält vorzugsweise eine kroykonservierte biologische Probe.

Das Verfahren umfasst ferner das gekühlte Lagern der Vorrichtung zur Kryokonservierung. Das Verfahren umfasst ferner das Überwachen des Füllstandes der mindestens einen Indikatorsubstanz in der mindestens einen Kammer.

Hat der Füllstand nach Beginn der Kryolagerung im Vergleich zum Anfangsfüllstand abgenommen, muss Indikatorsubstanz gasförmig aus der mindestens einen Öffnung entwichen sein. Folglich kann auf ein Überschreiten der Siede- oder Sublimationstemperatur und somit der zu überwachenden Grenztemperatur geschlossenen werden, insbesondere auch dann, wenn die Überschreitung nur kurzzeitig aufgetreten ist.

Ein besonderer Vorzug der Erfindung liegt somit darin, dass der aktuelle Füllstand der Indikatorsubstanz in der Kammer direkt anzeigt, ob eine Kryoprobe sich über eine definierbare Grenztemperatur, wenn auch nur kurzzeitig, erwärmt hat. Dies kann durch visuelle Sichtprüfung oder auch technisch automatisiert mittels einer entsprechend eingerichteten Messeinrichtung schnell und einfach festgestellt werden, ohne dass die Probe aus dem Probenbehälter entnommen oder aufgetaut werden muss.

Gemäß einer vorteilhaften Weiterbildung des Verfahrens kann ferner eine Kenngröße bestimmt werden, die eine Veränderung der Menge an Indikatorsubstanz in der jeweiligen Kammer angibt und/oder die ein Maß für die Dauer, die die Probe bei einer Temperatur oberhalb des Siedepunkts oder des Sublimationspunkts der Indikatorsubstanz verbracht hat, angibt.

Die zuvor beschriebenen bevorzugten Ausführungsformen und Merkmale der Erfindung sind miteinander kombinierbar. Weitere Einzelheiten und Vorteile der Erfindung werden im Folgenden unter Bezug auf die beigefügten Zeichnungen beschrieben. Es zeigen:
- FIG. 1A - 1C: schematische Ansichten eines ersten Ausführungsbeispiels einer Vorrichtung zur Temperaturüberwachung einer kryokonservierten biologischen Probe;
- FIG. 1D: eine schematische Ansicht einer Variante des ersten Ausführungsbeispiels;
- FIG. 2: schematische Ansichten eines zweiten Ausführungs-beispiels einer Vorrichtung zur Temperaturüberwachung einer kryokonservierten biologischen Probe; und
- FIG. 3: ein Ablaufdiagramm zur Illustration eines Ausführungsbeispiels eines Verfahrens zur Temperaturüberwachung einer kryokonservierten biologischen Probe.

Gleiche oder funktional äquivalente Elemente sind in allen Figuren mit denselben Bezugszeichen bezeichnet und werden zum Teil nicht gesondert beschrieben.

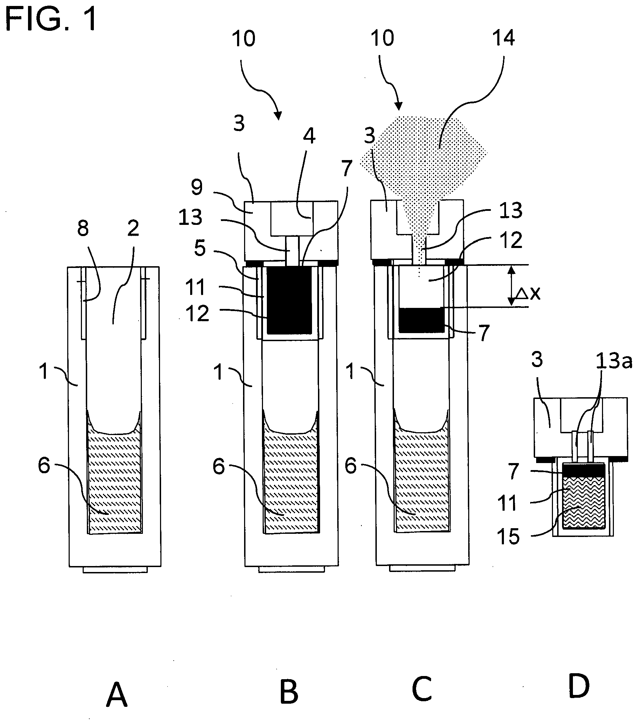

Gezeigt ist in Figur 1A beispielhaft ein offenes Kryoröhrchen 1 in einer Schnittansicht, stellvertretend für andere Kryoprobenbehälter, wie Straws, Beutel, Boxen etc. Derartige Kryoröhrchen 1 werden auch als Biobankröhrchen bezeichnet.

Das Kryoröhrchen 1 umfasst ein Aufnahmevolumen 2 für die Bioprobe, in dem sich bei der Verwendung der Vorrichtung die Biomaterialien befinden. Die Bioprobe ist hier eine Zellsuspension 6. Das Kryoröhrchen ist in Figur 1A noch ohne Schraubdeckelverschluss gezeigt. Das Kryoröhrchen umfasst einen in Figur 1B gezeigten Deckel (Schraubdeckelverschluss) 3, der das Kryoröhrchen 1 verschließt und oben einen Eingriff 4 besitzt, über den der Deckel 3 mit einem Werkzeug (nicht gezeigt) im Fall der Automatisierung gedreht werden kann. Diese Kryoröhrchen 1 können auch einen Boden enthalten, in den optional ein Barcodeviereck oder eine andere Kennung eingefügt ist. In dieser Form, zumeist senkrecht in Aufnahmen stehend, werden die Kryoröhrchen 1 in den Tieftemperaturbehältern gelagert.

In Figur 1B ist die für die Kryolagerung lagerbereite Vorrichtung 10 dargestellt. Der Deckel 3 hat ein auf dem Aufnahmevolumen 2 aufsitzendes Kopfteil 9 und einen daran angeformten Schaft 5, der in das Aufnahmevolumen 2 im verschraubten Zustand eingreift. Der korrespondierende Bereich des Aufnahmevolumens 2 weist ein Gewinde 8 auf. In den Schraubdeckel 3 ist eine Kammer 11 integriert, die ein Testvolumen ausbildet.

Die Kammer 11 weist eine oder mehrere offenen Verbindungen 13 zum Außenraum auf und hat keine Verbindung zum Aufnahmeraum 2 des Kryoröhrchens, in dem sich die Bioprobe 6 befindet.

Die Kammer 11 wird separat abgekühlt und mit der Indikatorsubstanz 7 befüllt. Das kann z.B. über Durchströmen mit CO₂-Gas oder Lachgas erfolgen, das in der Kammer 11 aufgrund der niedrigen Temperatur (< -140 °C) flüssig oder fest wird. Ist das Volumen 12 der Kammer 11 teilweise oder vollständig befüllt, wird das Kryoröhrchen 1 mit dem Deckel 3 verschlossen, wie in Figur 1B gezeigt. Die Füllung aus Indikatorsubstanz 7 ist durch die schwarze Fläche dargestellt.

Wird die Probe 6 im Fall von Trockeneis als Indikatorsubstanz 7 unberechtigterweise bei einer Temperatur über -78,5 °C gelagert, sublimiert das feste CO₂ und entweicht über die Öffnung 13 in die Umgebung, was durch das Bezugszeichen 14 dargestellt ist. Dadurch nimmt mit der Zeit der Pegelstand der Füllung um die Differenz Δx ab. Am Stand der Kammerfüllung kann sowohl die Verletzung der Grenztemperatur als auch in gewissem Umfang die Zeit der Fehllagerung bestimmt werden. Dies ist in Figur 1C dargestellt. Hierzu kann die Testsubstanz auch in geeigneter Weise eingefärbt oder über Sensoren in ihrer Menge bestimmt werden.

Die Vorrichtung 10, gebildet aus Probenbehälter 1 und integrierter Kammer 11, die mit Indikatorsubstanz 7 gefüllt ist, ist somit zur Temperaturüberwachung einer kryokonservierten biologischen Probe ausgelegt.

Gemäß einer weiteren Variante, die in Figur 1D dargestellt ist, kann der Innenraum der Kammer 11 auch partiell unten mit einem porösen Material 15 gefüllt sein, in das eine Flüssigkeit langsam hineindiffundiert. Wird z. B. statt CO₂ Lachgas (N₂O) als Indikatorsubstanz 7 durch das kalte Volumen der Kammer 11 geleitet, dann füllt sich der Raum über dem porösen Material, wie in Figur 1D gezeigt, mit Indikatorsubstanz 7 und wird bei einer Temperatur von < -140 °C fest. Wird nun die Vorrichtung 10 später in senkrechter Lage über die Schmelztemperatur -90,86 °C erwärmt und längere Zeit gelagert, diffundiert das flüssige Lachgas in den porösen Stoff hinein, was quantitativ abgelesen werden kann. Liegt die Lagertemperatur oberhalb -88,46 °C, verdunstet das Lachgas über die Öffnungen 13a in die Umgebung. Dann ist die Abnahme des Lachgaspegels das Maß und auch das poröse Material frei von diesem. Aus diesen Kombinationsbefunden lassen sich weit mehr Information über die Lagertemperatur gewinnen als über ein reines Schmelzen einer Substanz. Zudem ist der Verlust des Materials bei besonderer Zusammensetzung fälschungssicher. In geeigneter Weise kann ein Wiederbefüllen auch unterbunden werden.

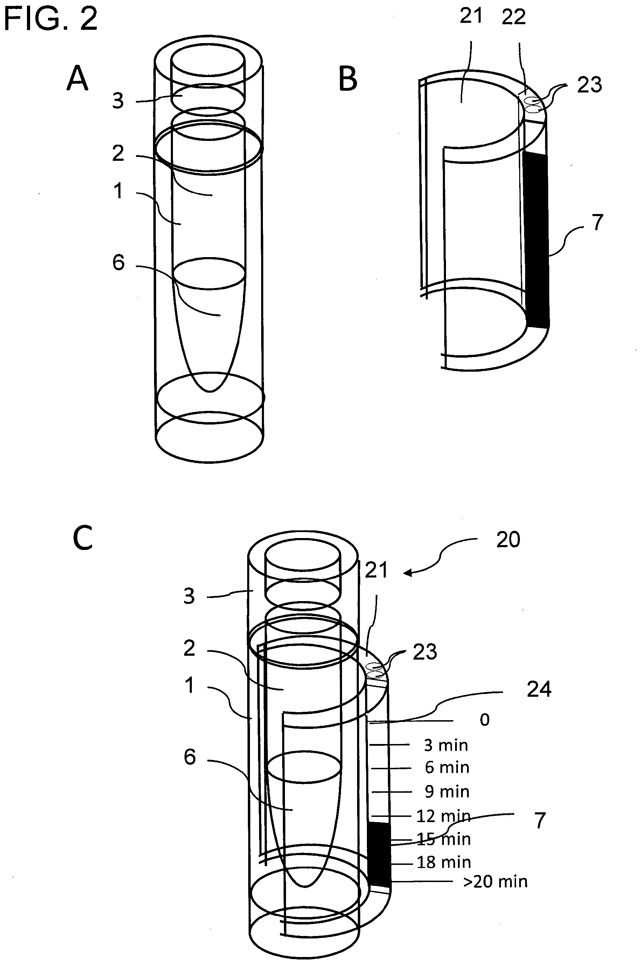

Figur 2A zeigt zunächst ein mit einem Deckel 3 komplett verschlossenes Kryoröhrchen 1, wie es üblicherweise in Kryobanken verwendet wird. Figur 2B zeigt ein doppelwandig ausgeführtes Aufsteckteil 21 aus Plastik, das auf die äußere Mantelfläche des Kryoröhrchens 1 aufgesetzt werden kann, wie in Figur 2C dargestellt.

In diesem Plastikteil 21 befindet sich analog zu dem in Figur 1 illustrierten Ausführungsbeispiel in senkrechter Ausrichtung ein Volumen 22, das mit einer Indikatorsubstanz 7 gefüllt ist. Das Volumen weist zwei Öffnungen 23 auf, über die ein Gas (z. B. CO₂-Gas oder Lachgas) oder eine Flüssigkeit eingeleitet werden kann.

Dieses Zusatzteil 21 kann auf bereits vorhandene Kryoröhrchen 1, wie in Figur 1C gezeigt, aufgeschoben und verklemmt werden. Das erfolgt bei der Lagertemperatur, in der Regel unter -140 °C, so dass die Indikatorsubstanz 7 im Aufsteckteil 21 flüssig oder fest ist und nicht über die Öffnungen 23 entweichen kann.

Analog zu dem in Figur 1 illustrierten Ausführungsbeispiel entweicht die Indikatorsubstanz 7 erst dann über die Öffnungen 23 aus dem Innenraum 22, wenn eine Siede- oder Sublimationstemperatur der Indikatorsubstanz 7 überschrittet wurde und die Indikatorsubstanz 7 in den gasförmigen Aggregatszustand übergeht.

Figur 2C zeigt einen Zustand, bei dem die Indikatorsubstanz 7 bereits größtenteils aus dem Innenraum 22 entwichen ist.

Die Wandung des Aufsteckteils weist eine Skala 24 auf und ist zumindest an dieser Stelle transparent ausgeführt, so dass der Füllstand an Indikatorsubstanz 7 von außen einsehbar ist. Über die Skala 24 kann die Abnahme der Indikatorsubstanz 7 noch zeitlich aufgelöst erfasst werden. In analoger Weise können solche Systeme auch an Boxen, Kästen, Straws und Beuteln oder anderen üblichen Kryobehältern befestigt werden.

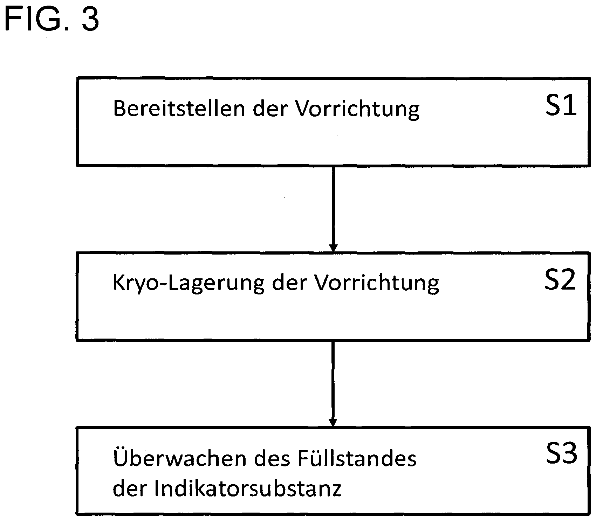

Figur 3 illustriert anhand eines Ablaufdiagramms ein Verfahren zur Temperaturüberwachung einer kryokonservierten biologischen Probe. In Schritt S1 wird eine erfindungsgemäße Vorrichtung zur Temperaturüberwachung bereitgestellt, beispielsweise eine der Vorrichtungen 10 oder 20, deren Kammer mit der Indikatorsubstanz gefüllt ist. Hierbei ist je nach Temperaturgrenzwert, der bei der Kryolagerung überwacht werden soll, eine Substanz, die einen geeigneten Siedepunkt oder Sublimationspunkt aufweist, als Indikatorsubstanz 7 auszuwählen.

In Schritt S2 wird die Vorrichtung mit einer Kryoprobe im Aufnahmeraum des Probenbehälters bei einer Lagertemperatur unterhalb der Siedetemperatur bzw. der Sublimationstemperatur der Indikatorsubstanz gelagert.

Nachfolgend kann mittels der Indikatorsubstanz zu einem beliebigen Zeitpunkt während des Lagervorgangs überprüft werden, ob eine unerwünschte, wenn auch nur zeitweise Erwärmung der Kryoprobe stattgefunden hat (Schritt S3). Hierzu wird jeweils der Füllstand der Kammer geprüft, d. h. ob sich und ggfs. wie stark sich der Füllstand an Indikatorsubstanz im Vergleich zum Ausgangszustand geändert hat. Wird zu einem späteren Kontrollzeitpunkt festgestellt, dass die Menge an fester oder flüssiger Indikatorsubstanz in der Kammer abgenommen hat, kann auf eine zwischenzeitlich erfolgte, zumindest kurzzeitige Überschreitung der Grenztemperatur geschlossen werden. Die Kammer kann daher als Indikatorelement bzw. Indikatoreinrichtung verwendet werden, um eine unerwünschte Überschreitung der Grenztemperatur anzuzeigen.

Obwohl die Erfindung unter Bezugnahme auf bestimmte Ausführungsbeispiele beschrieben worden ist, ist es für einen Fachmann ersichtlich, dass verschiedene Änderungen ausgeführt werden können, ohne den Bereich der Erfindung zu verlassen. Folglich soll die Erfindung nicht auf die offenbarten Ausführungsbeispiele begrenzt sein, sondern soll alle Ausführungsbeispiele umfassen, die in den Bereich der beigefügten Patentansprüche fallen.

## Patentansprüche

1. Vorrichtung (10; 20) zur Temperaturüberwachung einer kryokonservierten biologischen Probe, umfassend
a) einen Probenbehälter (1) mit einem Aufnahmeraum (2) zur Aufnahme einer biologischen Probe (6); und
b) mindestens eine Kammer (11; 21), deren Innenraum (12; 22) mit dem Aufnahmeraum nicht fluidisch verbunden ist und zumindest zum Teil mit einer Indikatorsubstanz (7) gefüllt ist, deren Siedetemperatur oder Sublimationstemperatur in einem Bereich von -10 °C bis -140 °C liegt, wobei die Kammer (11; 21) mindestens eine Öffnung (13; 23) aufweist, über die die Indikatorsubstanz (7) bei Überschreitung ihrer Siedepunkts oder ihres Sublimationspunkts aus dem Innenraum (12; 22) der Kammer (11; 21) entweichen kann.

2. Vorrichtung nach Anspruch 1, **gekennzeichnet durch** eine Mehrzahl von Kammern, die jeweils zumindest zum Teil mit einer Indikatorsubstanz, deren Siedetemperatur oder Sublimationstemperatur in einem Bereich von -10 °C bis -140 °C liegt, gefüllt sind, wobei die Indikatorsubstanzen in den Kammern unterschiedliche Siedetemperaturen oder Sublimationstemperaturen aufweisen.

3. Vorrichtung nach Anspruch 1 oder 2, **dadurch gekennzeichnet, dass** eine Wandung der mindestens einen Kammer an mindestens einer Stelle transparent oder semi-transparent ist.

4. Vorrichtung nach einem der vorhergehenden Ansprüche, **dadurch gekennzeichnet, dass** eine Wandung der mindestens einen Kammer eine Skala (24) zur Anzeige eines Füllstands der Indikatorsubstanz im Innenraum und/oder eine Skala zur Anzeige einer Zeitdauer der Überschreitung des Siedepunkts oder des Sublimationspunkts der Indikatorsubstanz (7) aufweist.

5. Vorrichtung nach einem der vorhergehenden Ansprüche, **gekennzeichnet durch** eine Messeinrichtung, die ausgebildet ist, einen Füllstand der Indikatorsubstanz im Innenraum der mindestens einen Kammer zu bestimmen.

6. Vorrichtung nach einem der vorhergehenden Ansprüche, **dadurch gekennzeichnet, dass** die Indikatorsubstanz (7)
a) Lachgas (N₂0), Trockeneis (CO₂) oder Ammoniak (NH₃) ist; und/oder
b) eingefärbt ist.

7. Vorrichtung nach einem der vorhergehenden Ansprüche, **dadurch gekennzeichnet, dass** die Öffnung über eine Kapillare gebildet wird, über die der Innenraum mit der Umgebung fluidisch in Verbindung steht.

8. Vorrichtung nach einem der vorhergehenden Ansprüche, **dadurch gekennzeichnet, dass** in einem Teilbereich der Kammer, in den die Öffnung nicht mündet, ein Material mit flüssigkeitsaufnehmender Struktur (15), beispielsweise ein poröses Material, vorhanden ist.

9. Vorrichtung nach einem der vorhergehenden Ansprüche, **dadurch gekennzeichnet, dass** die mindestens eine Kammer im Innern des Probenbehälters, insbesondere im Aufnahmeraum und/oder im Deckel (3), angeordnet ist.

10. Vorrichtung nach einem der vorhergehenden Ansprüche, **dadurch gekennzeichnet,**
a) **dass** der Probenbehälter (1) ein Kryoröhrchen ist, aufweisend einen Deckel (3) zum Verschließen des Aufnahmeraums, der einen mit einem oberen Endbereich des Aufnahmeraums in Eingriff stehenden Schaft (5) aufweist,
b) **dass** die mindestens eine Kammer (11) in den Schaft (5) integriert ist; und
c) **dass** ein auf dem Schaft sitzendes Kopfteil (9) des Deckels zur Ausbildung der mindestens einen Öffnung eine Durchgangsöffnung (13) aufweist.

11. Vorrichtung nach einem der Ansprüche 1 bis 9, **dadurch gekennzeichnet, dass** die mindestens eine Kammer durch einen Behälter (21) gebildet ist, der außen am Probenbehälter anordenbar und/oder angeordnet ist.

12. Vorrichtung nach einem der Ansprüche 1 bis 8, **dadurch gekennzeichnet, dass** die mindestens eine Kammer durch einen Behälter (21) gebildet ist, der auf eine äußere Mantelfläche des Probenbehälters aufsteckbar ist und diese im aufgesteckten Zustand dabei zumindest teilweise umgreift.

13. Verfahren zur Temperaturüberwachung von kryokonservierten Proben, umfassend die Schritte:
a) Bereitstellen einer Vorrichtung (10; 20) zur Temperaturüberwachung nach einem der vorhergehenden Ansprüche, die mindestens eine Indikatorsubstanz im gefrorenen Zustand in der mindestens einen Kammer enthält, wobei der Aufnahmeraum eine kroykonservierte Probe enthält;
b) gekühltes Lagern des Vorrichtung zur Kryokonservierung;
c) Überwachen des Füllstandes der mindestens einen Indikatorsubstanz in der mindestens einen Kammer (11; 21).

14. Verfahren nach Anspruch 13, **dadurch gekennzeichnet, dass** eine Kenngröße bestimmt wird, die eine Veränderung der Menge an Indikatorsubstanz in der jeweiligen Kammer angibt und/oder die ein Maß für die Dauer, die die Probe bei einer Temperatur oberhalb des Siedepunkts oder des Sublimationspunkts der Indikatorsubstanz (7) verbracht hat, angibt.

15. Verfahren nach Anspruch 13 und 14, **dadurch gekennzeichnet, dass** eine Substanz als Indikatorsubstanz ausgewählt wird, deren Siede- oder Sublimationstemperatur einer vorbestimmten Grenztemperatur, deren Überschreiten überwacht werden soll, entspricht.

## Claims

1. Device (10; 20) for temperature monitoring of a cryopreserved biological sample, comprising
a) a sample container (1) with a receiving space (2) for receiving a biological sample (6); and
b) at least one chamber (11; 21), the inner space (12; 22) of which is not fluidically connected to the receiving space and is at least partially filled with an indicator substance (7), the boiling temperature or sublimation temperature of which lies in a range from -10°C to -140°C, wherein the chamber (11; 21) comprises at least one opening (13; 23) via which the indicator substance (7) can escape from the inner space (12; 22) of the chamber (11; 21) in the case of the exceeding of its boiling point or its sublimation point.

2. Device according to Claim 1, **characterised by** a plurality of chambers which are in each case at least partially filled with an indicator substance, the boiling temperature or sublimation temperature of which lies in a range from -10°C to -140°C, wherein the indicator substances in the chambers have different boiling temperatures or sublimation temperatures.

3. Device according to Claim 1 or 2, **characterised in that** a wall of the at least one chamber is transparent or semi-transparent at at least one point.

4. Device according to any one of the preceding claims, **characterised in that** a wall of the at least one chamber comprises a scale (24) to display a fill level of the indicator substance in the inner space and/or a scale to display a duration of the exceeding of the boiling point or the sublimation point of the indicator substance (7).

5. Device according to any one of the preceding claims, **characterised by** a measuring apparatus which is configured to determine a fill level of the indicator substance in the inner space of the at least one chamber.

6. Device according to any one of the preceding claims, **characterised in that** the indicator substance (7)
a) is nitrous oxide (N₂O), dry ice (CO₂) or ammonia (NH₃); and/or
b) is coloured.

7. Device according to any one of the preceding claims, **characterised in that** the opening is formed by a capillary via which the inner space is fluidically connected to the surroundings.

8. Device according to any one of the preceding claims, **characterised in that** a material with a liquid-absorbing structure (15), for example, a porous material, is present in a subregion of the chamber into which the opening does not discharge.

9. Device according to any one of the preceding claims, **characterised in that** the at least one chamber is arranged in the interior of the sample container, in particular in the receiving space and/or in the cover (3).

10. Device according to any one of the preceding claims, **characterised in that**
a) the sample container (1) is a cryogenic tube, having a cover (3) for closing off the receiving space which has a shaft (5) which is in engagement with an upper end region of the receiving space,
b) the at least one chamber (11) is integrated into the shaft (5); and
c) a head part (9), which sits on the shaft, of the cover has a passage opening (13) for the formation of the at least one opening.

11. Device according to any one of Claims 1 to 9, **characterised in that** the at least one chamber is formed by a container (21) which can be arranged and/or is arranged on the outside of the sample container.

12. Device according to any one of Claims 1 to 8, **characterised in that** the at least one chamber is formed by a container (21) which can be pushed onto an outer shell surface of the sample container and at least partially engages around it in the pushed-on state.

13. Method for temperature monitoring of cryopreserved samples, comprising the steps:
a) providing a device (10; 20) for temperature monitoring according to any one of the preceding claims, which contains at least one indicator substance in the frozen state in the at least one chamber, wherein the receiving space contains a cryopreserved sample;
b) cooled storing of the device for cryopreservation;
c) monitoring the fill level of the at least one indicator substance in the at least one chamber (11; 21).

14. Method according to 13, **characterised in that** a parameter is determined which indicates a change in the quantity of indicator substance in the respective chamber and/or which indicates a measure for the period of time which the sample has spent at a temperature above the boiling point or the sublimation point of the indicator substance (7).

15. Method according to Claim 13 and 14, **characterised in that** a substance is selected as the indicator substance, the boiling or sublimation temperature of which corresponds to a predetermined threshold temperature, the exceeding of which should be monitored.

## Revendications

1. Dispositif (10 ; 20) pour la surveillance de la température d'un échantillon biologique cryconservé, comprenant
a) un récipient d'échantillons (1) avec un espace de réception (2) pour recevoir un échantillon (6) biologique ; et
b) au moins un compartiment (11 ; 21), dont l'espace intérieur (12 ; 22) n'est pas relié de manière fluidique à l'espace de réception et est rempli au moins en partie avec une substance indicatrice (7), dont la température d'ébullition ou la température de sublimation se situe dans une plage de -10 °C à -140 °C, dans lequel le compartiment (11 ; 21) présente au moins une ouverture (13 ; 23), par l'intermédiaire de laquelle la substance indicatrice (7) peut s'échapper hors de l'espace intérieur (12 ; 22) du compartiment (11 ; 21) en cas de dépassement de son point d'ébullition ou de son point de sublimation.

2. Dispositif selon la revendication 1, **caractérisé par** une multitude de compartiments, qui sont remplis respectivement au moins en partie d'une substance indicatrice, dont la température d'ébullition ou la température de sublimation se situe dans une plage de-10 °C à -140 °C, dans lequel les substances indicatrices présentent dans les compartiments des températures d'ébullition ou des températures de sublimation différentes.

3. Dispositif selon la revendication 1 ou 2, **caractérisé en ce qu'**une paroi de l'au moins un compartiment est transparente ou semi-transparente sur au moins un emplacement.

4. Dispositif selon l'une quelconque des revendications précédentes, **caractérisé en ce qu'**une paroi de l'au moins un compartiment présente une graduation (24) pour indiquer un niveau de remplissage de la substance indicatrice dans l'espace intérieur et/ou une graduation pour indiquer une durée du dépassement du point d'ébullition ou du point de sublimation de la substance indicatrice (7).

5. Dispositif selon l'une quelconque des revendications précédentes, **caractérisé par** un système de mesure qui est réalisé pour définir un niveau de remplissage de la substance indicatrice dans l'espace intérieur de l'au moins un compartiment.

6. Dispositif selon l'une quelconque des revendications précédentes, **caractérisé en ce que** la substance indicatrice (7)
a) est un gaz hilarant (N₂O), de la neige carbonique (CO₂) ou de l'ammoniaque (NH₃) ; et/ou
b) est colorée.

7. Dispositif selon l'une quelconque des revendications précédentes, **caractérisé en ce que** l'ouverture est formée par l'intermédiaire d'un capillaire, par l'intermédiaire duquel l'espace intérieur est relié de manière fluidique à l'environnement.

8. Dispositif selon l'une quelconque des revendications précédentes, **caractérisé en ce qu'**est présent, dans une zone partielle du compartiment, dans laquelle l'ouverture ne débouche pas, un matériau avec une structure d'absorption de liquide (15), par exemple un matériau poreux.

9. Dispositif selon l'une quelconque des revendications précédentes, **caractérisé en ce que** l'au moins un compartiment est disposé à l'intérieur du récipient d'échantillons, en particulier dans l'espace de réception et/ou dans le couvercle (3).

10. Dispositif selon l'une quelconque des revendications précédentes, **caractérisé en ce**
a) **que** le récipient d'échantillons (1) est un cryotube, présentant un couvercle (3) pour fermer l'espace de réception, qui présente une tige (5) se trouvant en prise avec une zone d'extrémité supérieure de l'espace de réception,
b) **que** l'au moins un compartiment (11) est intégré dans la tige (5) ; et
c) **qu'**une partie tête (9), siégeant dans la tige, du couvercle présente, pour réaliser l'au moins une ouverture, une ouverture de passage (13).

11. Dispositif selon l'une quelconque des revendications 1 à 9, **caractérisé en ce que** l'au moins un compartiment est formé par un récipient (21), qui peut être disposé et/ou est disposé à l'extérieur sur le récipient d'échantillons.

12. Dispositif selon l'une quelconque des revendications 1 à 8, **caractérisé en ce que** l'au moins un compartiment est formé par un récipient (21), qui peut être emboîté sur une surface enveloppante extérieure du récipient d'échantillons et entoure ce faisant au moins en partie celle-ci dans l'état emboîté.

13. Procédé pour la surveillance de la température d'échantillons cryoconservés, comprenant les étapes :
a) de fourniture d'un dispositif (10 ; 20) pour la surveillance de la température selon l'une quelconque des revendications précédentes, qui contient au moins une substance indicatrice dans l'état gelé dans l'au moins un compartiment, dans lequel l'espace de réception contient un échantillon cryoconservé ;
b) d'entreposage réfrigéré du dispositif pour la cryoconservation ;
c) de surveillance du niveau de remplissage de l'au moins une substance indicatrice dans l'au moins un compartiment (11 ; 21).

14. Procédé selon la revendication 13, **caractérisé en ce qu'**est définie une grandeur caractéristique, qui indique une modification de la quantité en substance indicatrice dans le compartiment concerné et/ou qui indique une valeur pour la durée passée par l'échantillon à une température supérieure au point d'ébullition ou au point de sublimation de la substance indicatrice (7).

15. Procédé selon les revendications 13 et 14, **caractérisé en ce qu'**une substance est choisie en tant que substance indicatrice, dont la température d'ébullition ou de sublimation correspond à une température limite prédéfinie, dont le dépassement doit être surveillé.
